# EUROPEAN PATENT APPLICATION

(11) **EP 3 192 802 A1**
(43) Date of publication of application: **19.07.2017**
(21) Application number: 17152060.4
(22) Date of filing: 18.01.2017
(51) Int. Cl.: C07K 7/08, C12N 9/64

(54) **STABILIZED PEPTIDE DERIVATIVES**

(30) Priority: 18.01.2016 GB 201600911
(71) Applicant: Bicycle Therapeutics Limited, Babraham Research Campus Cambridge CB22 3AT (GB)
(72) Inventor: Teufel, Daniel, Cambridge, Cambridgeshire CB22 3AT (GB); Baldassarre, Leonardo, Cambridge, Cambridgeshire CB22 3AT (GB); Mudd, Gemma, Cambridge, Cambridgeshire CB22 3AT (GB); Pavan, Silvia, Cambridge, Cambridgeshire CB22 3AT (GB)
(74) Representative: Maschio, Antonio

(57) **Abstract**

A compound comprising at least one looped peptide structure attached via at least two sulfone linkages formed through cysteine residues of the peptide to a scaffold, wherein the looped peptide comprises an amino acid sequence of formula (I):

-Cᵢ-X-U/O-X-X-G-Cᵢᵢ-E-D-F-Y-X-X-Cᵢᵢᵢ- (SEQ ID NO: 1) (I)

or a modified derivative, or pharmaceutically acceptable salt, thereof;
wherein:
Cᵢ, Cᵢᵢ and Cᵢᵢᵢ represent first, second and third cysteine residues, respectively;
X represents any amino acid residue;
U represents a polar, uncharged amino acid residue selected from N, C, Q, M, S and T; and
O represents a non-polar aliphatic amino acid residue selected from G, A, I, L, P and V.

Preferably, the compound is a bicyclic peptide having two peptide loops between three sulfone linkages to the scaffold.

## Description

### Technical Field

The present invention relates to peptides whose structure is constrained by binding to a scaffold moiety which provides a structural backbone, imparting a conformation to the peptide. In particular, the invention relates to novel chemistries for forming two or more bonds between the peptide and a scaffold molecule.

### Background of the Invention

Different research teams have previously tethered peptides to scaffold moieties by forming two or more thioether bonds between cysteine residues of the peptide and suitable functional groups of a scaffold molecule. For example, methods for the generation of candidate drug compounds by linking cysteine-containing peptides to a molecular scaffold as for example tris(bromomethyl)benzene are disclosed in WO2004/077062 and WO2006/078161.

Copending PCT International Patent Application of the present applicant with title "Bicyclic Peptide Ligands Specific for MT1-MMP" filed October 2015 under attorney reference BIC-C-P1803PCT, the entire content of which is incorporated herein by reference, describes thioether-linked looped peptide structures of the type disclosed in WO2004/077062 and WO2006/078161, wherein the peptide sequences are selected to provide selective binding to the membrane metalloprotease MT1-MMP.

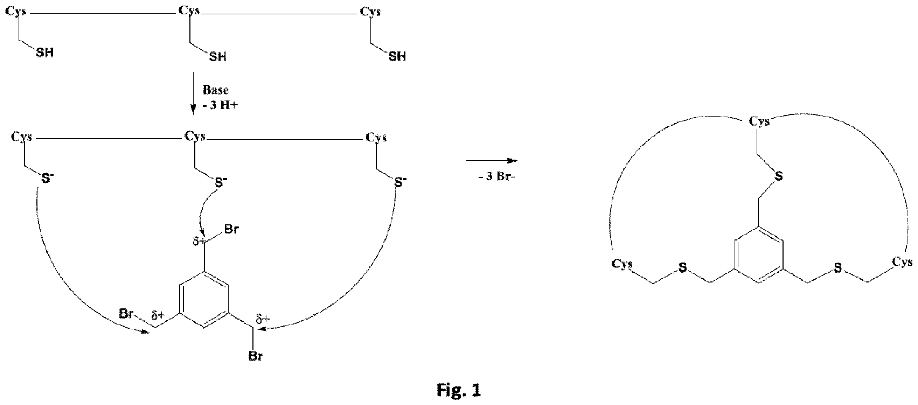

The advantage of utilising cysteine thiols for generating covalent thioether linkages in order to achieve cyclisation resides is their selective and biorthogonal reactivity. Thiol-containing Bicyclic peptides may be cyclised with a thiol-reactive scaffold compound such as 1, 3, 5 tris-bromomethylbenzene (TBMB),and the resultant product contains three thioethers at the benzylic locations. The overall reaction of the linear peptide with TBMB to form a looped bicyclic peptide with thioether linkages is shown in Fig. 1.

WO2011/018227 describes methods for altering the conformation of bicyclic peptide derivatives of the above type. The disclosed methods include: (a) altering at least one reactive group of the peptide used to make the derivative; or (b) altering the nature of the molecular scaffold; or (c) altering the bond between at least one reactive group and the molecular scaffold; or any combination of (a), (b) or (c). The specification describes *inter alia* oxidizing the thioether bond between the scaffold and the peptide to form a sulfone bond between them. However, in the example given, this oxidation results in almost complete loss of the measured binding affinity of a PK15-TBMB conjugate towards kallikrein. It is suggested that this loss of activity is due to conformational changes induced by the oxidation of the thioether linkage.

In certain *in vivo* applications, and in the course of the manufacture of such thioether bonded peptides, the sulfur may represent a group that is susceptible to oxidation, forming in the first instance an *R* or *S* sulfoxide, and upon further oxidation, a sulfone:

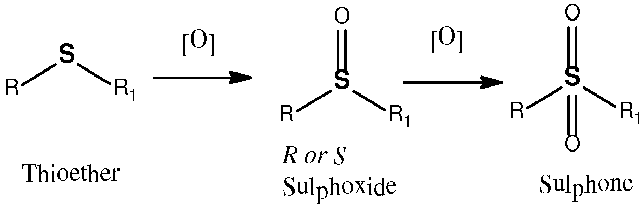

In the *in vivo* setting, these oxidised species may 1) alter the specificity of the (bicyclic) peptide to its target and its homologues, 2) unfavourably lower the affinity of the peptide to its target, lowering exposure, 3) complicate the interpretation pharmacokinetics and pharmacodynamics *in vivo*, and 4) complicate the bioanalytical quantification *in vivo*.

In the manufacturing setting, the thioethers (as well as the thiol starting material) are sensitive to oxygen in the air, and special precautions are required to minimise formation of these oxidised species. Oxidised products result in lower yields and complications during purification and workup. Furthermore, upon preparation and storage of the formulated drug product for in vivo applications, or other engineering applications, thioethers are once again sensitive to oxidative processes and various precautions such as specialised formulations and protective atmospheres may be necessary.

A need therefore exists for improved chemistries for coupling peptides to scaffold moieties to form looped peptide structures.

### Summary of the Invention

The present inventors have found that oxidation of the thioether linkages to sulfone linkages in certain looped peptide conjugates can be achieved substantially quantitatively, without substantial loss of the functional binding activity of the cyclised peptides. In particular, it appears that the above-identified bicyclic peptide specific for MT1-MMP have this property. This finding is surprising in view of the teachings of WO2011/018227, and offers a pathway to stabilized bicyclic peptide that have improved oxidation stability.

Accordingly, in a first aspect the present invention provides a compound comprising at least one looped peptide structure attached via at least two sulfone linkages formed through cysteine residues of the peptide to a scaffold, wherein the looped peptide comprises an amino acid sequence of formula (I):

-Cᵢ-X-U/O-X-X-G-Cᵢᵢ-E-D-F-Y-X-X-Cᵢᵢᵢ- (SEQ ID NO: 1) (I)

or a modified derivative, or pharmaceutically acceptable salt, thereof;
wherein:
Cᵢ, Cᵢᵢ and Cᵢᵢᵢ represent first, second and third cysteine residues, respectively;
X represents any amino acid residue;
U represents a polar, uncharged amino acid residue selected from N, C, Q, M, S and T; and
O represents a non-polar aliphatic amino acid residue selected from G, A, I, L, P and V.

Suitably, the X at position 1 is selected from any one of the following amino acids: Y, M, F or V, such as Y, M or F, in particular, Y or M, more particularly Y. Suitably, the U/O at position 2 is selected from a U, such as an N, or an O, such as a G.

Suitably, the X at position 3 is selected from U or Z, wherein U represents a polar, uncharged amino acid residue selected from N, C, Q, M, S and T and Z represents a polar, negatively charged amino acid residue selected from D or E, in particular the U at position 3 is selected from Q or the Z at position 3 is selected from E.

Suitably, the X at position 4 is selected from J, wherein J represents a non-polar aromatic amino acid residue selected from F, W and Y.

Suitably, the X at position 10 is selected from Z, wherein Z represents a polar, negatively charged amino acid residue selected from D or E, such as D. Suitably, the X at position 11 is selected from O, wherein O represents a non-polar aliphatic amino acid residue selected from G, A, I, L, P and V, such as I.

More suitably, the compound of formula (I) is a compound of formula (Ia):

-Cᵢ-Y/M/F/V-U/O-U/Z-J-G-Cᵢᵢ-E-D-F-Y-Z-O-Cᵢᵢᵢ- (SEQ ID NO: 6) (Ia)

wherein U, O, J and Z are as defined hereinbefore; or a compound of formula (Ib):

-Cᵢ-Y/M/F/V-N/G-E/Q-F-G-Cᵢᵢ-E-D-F-Y-D-I-Cᵢᵢᵢ- (SEQ ID NO: 7) (Ib);

or
a compound of formula (Ic):

-Cᵢ-Y/M/F-N/G-E/Q-F-G-Cᵢᵢ-E-D-F-Y-D-I-Cᵢᵢᵢ- (SEQ ID NO: 8) (Ic);

or
a compound of formula (Id):

-Cᵢ-Y/M-N-E/Q-F-G-Cᵢᵢ-E-D-F-Y-D-I-Cᵢᵢᵢ- (SEQ ID NO: 9) (Id);

or a compound of formula (Ie):
- Cᵢ-Y-N-E-F-G-Cᵢᵢ-E-D-F-Y-D-I-Cᵢᵢᵢ- (17-69-07) (SEQ ID NO: 2) (Ie).

For example, the peptide of formula (I) may comprise a sequence selected from:
-Cᵢ-Y-N-E-F-G-Cᵢᵢ-E-D-F-Y-D-I-Cᵢᵢᵢ- (17-69-07) (SEQ ID NO: 2);
-Cᵢ-M-N-Q-F-G-Cᵢᵢ-E-D-F-Y-D-I-Cᵢᵢᵢ- (17-69-12) (SEQ ID NO: 10);
-Cᵢ-F-G-E-F-G-Cᵢᵢ-E-D-F-Y-D-I-Cᵢᵢᵢ- (17-69-02) (SEQ ID NO: 11);
-Cᵢ-V-N-E-F-G-Cᵢᵢ-E-D-F-Y-D-I-Cᵢᵢᵢ- (17-69-03) (SEQ ID NO: 12);
-CᵢF-N-E-F-G-Cᵢᵢ-E-D-F-Y-D-I-Cᵢᵢᵢ- (17-69-04) (SEQ ID NO: 13);
-Cᵢ-Y-N-E-Y-G-Cᵢᵢ-E-D-F-Y-D-I-Cᵢᵢᵢ- (17-69-07-N057) (SEQ ID NO: 14); and
-Cᵢ-Y-N-E-W-G-Cᵢᵢ-E-D-F-Y-D-1-Cᵢᵢᵢ- (17-69-44-N002) (SEQ ID NO: 15),
such as:
-Cᵢ-Y-N-E-F-G-Cᵢᵢ-E-D-F-Y-D-I-Cᵢᵢᵢ- (17-69-07) (SEQ ID NO: 2); and
-Cᵢ-M-N-Q-F-G-Cᵢᵢ-E-D-F-Y-D-I-Cᵢᵢᵢ- (17-69-12) (SEQ ID NO: 10),
in particular:
-Cᵢ-Y-N-E-F-G-Cᵢᵢ-E-D-F-Y-D-I-Cᵢᵢᵢ- (17-69-07) (SEQ ID NO: 2).

Still more suitably, the peptide of formula (I) has sequence:
H-(b-Ala)-Sar₁₀-Ala-Cys-(D-Ala)-Asn-Glu-(1Nal)-(D-Ala)-Cys-Glu-Asp-Phe-Tyr-Asp-(tBuGly)-Cys-NH2 (SEQ ID NO: 5); or
Acetyl-(b-Ala)-Sar₁₀-Ala-Cys-(D-Ala)-Asn-Glu-(1Nal)-(D-Ala)-Cys-Glu-Asp-Phe-Tyr-Asp-(tBuGly)-Cys-NH2 (SEQ ID NO:); or
H-Ala-Cys-(D-Ala)-Asn-Glu-(1Nal)-(D-Ala)-Cys-Glu-Asp-Phe-(4BrPhe)-Asp-(tBuGly)-Cys-NH2 (SEQ ID NO:),
wherein Sar is sarcosine, where D-Ala is D-Alanine, where 1NAl is 1-naphthyl-L-alanine, where tBuGly is α-t-butylglycine and where b-Ala is β-Alanine.

Preferably, the looped peptide structure is attached to the scaffold via at least three sulfone linkages to form a bicyclic structure on the scaffold.

Suitably, the scaffold comprises a (hetero)aromatic or (hetero)alicyclic moiety. Suitably, the scaffold comprises a *tris*-substituted (hetero)aromatic or (hetero)alicyclic moiety, for example a tris-methylene substituted (hetero)aromatic or (hetero)alicyclic moiety. The (hetero)aromatic or (hetero)alicyclic moiety is suitably a six-membered ring structure, preferably *tris*-substituted such that the scaffold has a 3-fold symmetry axis. Thus, in embodiments, the scaffold is 1,3,5-*tris*-benzyl phenyl.

Suitably, the thioether linkages are made through cysteine amino acid residues of the peptide.

In a second aspect, the present invention provides a method of making a compound according to the present invention, the method comprising: providing a peptide having at least two cysteine residues; providing a scaffold molecule having at least two reactive sites for forming thioether linkages with the cysteine residues; forming said thioether linkages between the peptide and the scaffold molecule; and oxidizing the thioether linkages to sulfone linkages.

The suitable and preferred features of the peptide and the scaffold molecule are as described above in relation to the first aspect of the invention.

In a further aspect, the present invention provides a compound comprising at least one looped peptide structure attached via at least two sulfone linkages formed through cysteine residues of the peptide to a scaffold, wherein the Kd of the compound towards a predetermined target molecule is no more than about 10 times the Kd of the corresponding compound having thioether linkages in place of the sulfone linkages.

In alternative aspects of the invention Kd may be replaced by Ki or by IC50. Suitably, the Kd of the compound towards a predetermined target molecule is no more than about 20 times the Kd of the corresponding compound having thioether linkages in place of the sulfone linkages. Suitably, the claimed peptide compound having sulfone linkages has Kd and/or Ki with the predetermined target less than about 50nM.

In another aspect, the invention further provides a kit comprising at least a peptide derivative according to the present invention.

In a still further aspect, the present invention provides a composition comprising a peptide derivative of the present invention, and a pharmaceutically acceptable carrier, diluent or excipient.

Moreover, the present invention provides a method for the treatment of disease using a peptide derivative or a composition according to the present invention.

In a further aspect, the present invention provides a method for the diagnosis, including diagnosis of disease using a peptide derivative, or a composition according to the present invention. Thus in general the binding of an analyte to a peptide derivative may be exploited to displace an agent, which leads to the generation of a signal on displacement. For example, binding of analyte (second target) can displace an enzyme (first target) bound to the peptide derivative providing the basis for a binding assay, especially if the enzyme is held to the peptide derivative through its active site.

### Brief Description of the Drawings

Fig. 1 shows a reaction scheme for the production of a Bicycle peptide covalently bonded to a scaffold by thioether linkages;
Fig. 2 shows the results of fluorescence polarization competition experiments to determine the comparative affinity of three MT1-MMP binding bicyclic peptide compounds, in thioether and sulfone versions.
Fig. 3 shows the reaction scheme to obtain a tris-sulphone Bicyclic peptide from a tris-thioether peptide

### Detailed Description of the Invention

Unless defined otherwise, all technical and scientific terms used herein have the same meaning as commonly understood by those of ordinary skill in the art, such as in the arts of peptide chemistry, cell culture and phage display, nucleic acid chemistry and biochemistry. Standard techniques are used for molecular biology, genetic and biochemical methods (see Sambrook et al., Molecular Cloning: A Laboratory Manual, 3rd ed., 2001, Cold Spring Harbor Laboratory Press, Cold Spring Harbor, NY; Ausubel et al., Short Protocols in Molecular Biology (1999) 4th ed., John Wiley & Sons, Inc.), which are incorporated herein by reference.

The present invention provides compound comprising at least one looped peptide structure attached via at least two sulfone linkages formed through cysteine residues of the peptide to a scaffold, wherein the looped peptide comprises an amino acid sequence of formula (I):

-Cᵢ-X-U/O-X-X-G-Cᵢᵢ-E-D-F-Y-X-X-Cᵢᵢᵢ- (SEQ ID NO: 1) (I)

or a modified derivative, or pharmaceutically acceptable salt, thereof;
wherein:
Cᵢ, Cᵢᵢ and Cᵢᵢᵢ represent first, second and third cysteine residues, respectively;
X represents any amino acid residue;
U represents a polar, uncharged amino acid residue selected from N, C, Q, M, S and T; and
O represents a non-polar aliphatic amino acid residue selected from G, A, I, L, P and V.

Suitably, the covalent linkages between the peptide and the scaffold consist essentially of, or consist of, only sulfone linkages. This can be achieved, for example, by complete oxidation of thioether-linked peptide conjugates ("peptide ligands") as described further below. The compounds of the invention are thus considerably more oxidation-stable than the thioether-linked conjugates of the prior art.

The peptide of formula (I) is a peptide that is known to exhibit high levels of binding to MT1-MMP when conjugated to a scaffold through thioether linkages. Surprisingly, the present inventors have found that oxidation of the thioether linkages to sulfone linkages in conjugates ("peptide ligands") made from these peptides does not greatly reduce the binding affinity of the conjugates to MT1-MMP.

The peptides in the conjugates according to this aspect of the invention are suitably further defined as follows.

### Numbering

When referring to amino acid residue positions within compounds of formula (I), cysteine residues (Cᵢ, Cᵢᵢ and Cᵢᵢᵢ) are omitted from the numbering as they are invariant, therefore, the numbering of amino acid residues within the compound of formula (I) is referred to as below:
-Cᵢ-X₁-U/O₂-X₃-X₄-G₅-Cᵢᵢ-E₆-D₇-F₈-Y₉-X₁₀-X₁₁-Cᵢᵢᵢ- (SEQ ID NO: 1).

For the purpose of this part of the description, all bicyclic peptides are assumed to be cyclised with TBMB (1,3,5-tris(bromomethyl)benzene) yielding a tri-substituted 1,3,5-trismethylbenzene structure. Cyclisation with TBMB occurs on Cᵢ, Cᵢᵢ, and Cᵢᵢᵢ.

### Bicyclic Peptide Core Sequence

Each bicyclic peptide disclosed herein has been assigned a unique core sequence number which is defined as the amino acid sequence between the first N-terminal Cysteine (Cᵢ) and the last C-terminal Cysteine (Cᵢᵢᵢ). In the example of the identifier 17-69-07, the core sequence is CᵢYNEFGCᵢᵢEDFYDICᵢᵢᵢ (SEQ ID NO: 2), and is referred to as "17-69-07" or "(17-69-07)".

### Peptide Code

Certain bicyclic peptides disclosed herein have also been assigned a unique identifier using a peptide code, such as 17-69-07-N241, wherein N241 denotes a particular derivative of the 17-69-07 bicycle core sequence. Different derivatives of 17-69-07 have different N-numbers, i.e. N001, N002, Nxxx.

### Molecular Format

N- or C-terminal extensions to the bicycle core sequence are added to the left or right side of the core sequence, separated by a hyphen. For example, an N-terminal βAla-Sar10-Ala tail would be denoted as:
βAla-Sar10-A-(17-69-07)
and has the full sequence of βAla-Sar10-A-CYNEFGCEDFYDIC (SEQ ID NO: 3).

### Modifications

Non-natural amino acid substitutions within the bicycle core sequence are indicated after the Molecular Format description. For example, if Tyrosine 1 in 17-69-07 is substituted with D-Alanine, the description is (17-69-07) D-Ala1, and the full sequence would be described as C(D-Ala1)NEFGCEDFYDIC (SEQ ID NO: 4).

If an N-terminal or C-terminal tail is attached to a bicyclic peptide that also contains modifications to the core sequence, then, by using 17-69-07-N241 as an example, the Molecular Format description is:
βAla-Sar10-A-(17-69-07) DAla1 1NAl4 DAla5 tBuGly11.

The full amino acid sequence of 17-69-07-N241 is therefore:
βAla-Sar10-A- C(D-Ala)NE(1Nal)(D-Ala)CEDFYD(tBuGly)C (SEQ ID NO: 5).

A peptide ligand, as referred to herein, refers to a peptide covalently bound to a molecular scaffold. Typically, such peptides comprise two or more reactive groups (i.e. cysteine residues) which are capable of forming covalent bonds to the scaffold, and a sequence subtended between said reactive groups which is referred to as the loop sequence, since it forms a loop when the peptide is bound to the scaffold. In the present case, the peptides comprise at least three cysteine residues (referred to herein as Cᵢ, Cᵢᵢ and Cᵢᵢᵢ), and form at least two loops on the scaffold.

It will be appreciated by the skilled person that the X at positions 1, 3, 4, 10 and 11 of formula (I) may represent any amino acid following the results of the alanine scan (see Table 5) and selection outputs (Figure 8) which permits well tolerated substitutions at these positions.

In one embodiment, the X at position 1 of formula (I) is selected from any one of the following amino acids: Y, M, F or V. In a further embodiment, the X at position 1 of formula (I) is selected from Y, M or F. In a yet further embodiment, the X at position 1 of formula (I) is selected from Y or M. In a still yet further embodiment, the X at position 1 of formula (I) is selected from Y.

In one embodiment, the U/O at position 2 of formula (I) is selected from a U, such as an N. In an alternative embodiment, the U/O at position 2 of formula (I) is selected from an O, such as a G.

In one embodiment, the X at position 3 of formula (I) is selected from U or Z, wherein U represents a polar, uncharged amino acid residue selected from N, C, Q, M, S and T and Z represents a polar, negatively charged amino acid residue selected from D or E. In a further embodiment, the U at position 3 of formula (I) is selected from Q. In an alternative embodiment, the Z at position 3 of formula (I) is selected from E.

In one embodiment, the X at position 4 of formula (I) is selected from J, wherein J represents a non-polar aromatic amino acid residue selected from F, W and Y. In a further embodiment, the J at position 4 of formula (I) is selected from F. In alternative embodiment, the J at position 4 of formula (I) is selected from Y. In alternative embodiment, the J at position 4 of formula (I) is selected from W.

In one embodiment, the X at position 10 of formula (I) is selected from Z, wherein Z represents a polar, negatively charged amino acid residue selected from D or E. In one embodiment, the Z at position 10 of formula (I) is selected from D.

In one embodiment, the X at position 11 of formula (I) is selected from O, wherein O represents a non-polar aliphatic amino acid residue selected from G, A, I, L, P and V. In one embodiment, the O at position 11 of formula (I) is selected from I.

In one embodiment, the compound of formula (I) is a compound of formula (Ia):

-Cᵢ-Y/M/F/V-U/O-U/Z-J-G-Cᵢᵢ-E-D-F-Y-Z-O-Cᵢᵢᵢ- (SEQ ID NO: 6) (Ia);

wherein U, O, J and Z are as defined hereinbefore.

In one embodiment, the compound of formula (I) is a compound of formula (Ib):

-Cᵢ-Y/M/F/V-N/G-E/Q-F-G-Cᵢᵢ-E-D-F-Y-D-I-Cᵢᵢᵢ- (SEQ ID NO: 7) (Ib).

In one embodiment, the compound of formula (I) is a compound of formula (Ic):

-Cᵢ-Y/M/F-N/G-E/Q-F-G-Cᵢᵢ-E-D-F-Y-D-I-Cᵢᵢᵢ- (SEQ ID NO: 8) (Ic).

In one embodiment, the compound of formula (I) is a compound of formula (Id):

-Cᵢ-Y/M-N-E/Q-F-G-Cᵢᵢ-E-D-F-Y-D-I-Cᵢᵢᵢ- (SEQ ID NO: 9) (Id).

In one embodiment, the compound of formula (I) is a compound of formula (Ie):

-Cᵢ-Y-N-E-F-G-Cᵢᵢ-E-D-F-Y-D-I-Cᵢᵢᵢ- (17-69-07) (SEQ ID NO: 2) (Ie).

In a yet further embodiment, the peptide of formula (I) comprises a sequence selected from:
-Cᵢ-Y-N-E-F-G-Cᵢᵢ-E-D-F-Y-D-I-Cᵢᵢᵢ- (17-69-07) (SEQ ID NO: 2);
-Cᵢ-M-N-Q-F-G-Cᵢᵢ-E-D-F-Y-D-I-Cᵢᵢᵢ- (17-69-12) (SEQ ID NO: 10);
-Cᵢ-F-G-E-F-G-Cᵢᵢ-E-D-F-Y-D-I-Cᵢᵢᵢ- (17-69-02) (SEQ ID NO: 11);
-Cᵢ-V-N-E-F-G-Cᵢᵢ-E-D-F-Y-D-I-Cᵢᵢᵢ- (17-69-03) (SEQ ID NO: 12);
-Cᵢ-F-N-E-F-G-Cᵢᵢ-E-D-F-Y-D-I-Cᵢᵢᵢ- (17-69-04) (SEQ ID NO: 13);
-Cᵢ-Y-N-E-Y-G-Cᵢᵢ-E-D-F-Y-D-I-Cᵢᵢᵢ- (17-69-07-N057) (SEQ ID NO: 14); and
-Cᵢ-Y-N-E-W-G-Cᵢᵢ-E-D-F-Y-D-I-Cᵢᵢᵢ- (17-69-44-N002) (SEQ ID NO: 15).

The peptides of this embodiment were identified to be potent candidates following affinity maturation against the hemopexin domain of MT1-MMP (see Example 1 and Tables 1 and 8).

In a still yet further embodiment, the peptide of formula (I) comprises a sequence selected from:
-Cᵢ-Y-N-E-F-G-Cᵢᵢ-E-D-F-Y-D-I-Cᵢᵢᵢ- (17-69-07) (SEQ ID NO: 2); and
-Cᵢ-M-N-Q-F-G-Cᵢᵢ-E-D-F-Y-D-I-Cᵢᵢᵢ- (17-69-12) (SEQ ID NO: 10).

The thioether bicycle peptides of this embodiment have been identified to be the highest affinity candidates following affinity maturation against the hemopexin domain of MT1-MMP, synthesis of the core bicycle sequences, and quantitative measurement of affinities using competition experiments.

In a still yet further embodiment, the peptide of formula (I) comprises a sequence selected from -Cᵢ-Y-N-E-F-G-Cᵢᵢ-E-D-F-Y-D-I-Cᵢᵢᵢ- (17-69-07) (SEQ ID NO: 2). The peptide of this embodiment was identified to be the most potent, and stable member of the family of peptide ligands within formula (I) .

In a yet further embodiment, the peptide ligand of the invention is selective for MT1-MMP, but does not cross-react with MMP-1, MMP-2, MMP-15 and MMP-16. It has been found that the 17-69-07 core sequence, and the stabilised variant 17-69-07-N258, are uniquely selective for MT1-MMP.

The compounds of the invention comprise, consist essentially of, or consist of, a peptide covalently bound to a molecular scaffold. The term "scaffold" or "molecular scaffold" in relation to this aspect and all other aspects of the invention refers to a chemical moiety that is bonded to the peptide at the two or more sulfone linkages in the compounds of the invention. The term "scaffold molecule" or "molecular scaffold molecule" herein refers to a molecule that is capable of being reacted with a peptide or peptide derivative to form the conjugate having sulfone bonds, or having thioether bonds for subsequent oxidation to sulfone. Thus, the scaffold molecule has the same structure as the scaffold moiety except that respective reactive groups (such as leaving groups) of the molecule are replaced by sulfone bonds to the peptide in the scaffold moiety.

The molecular scaffold molecule is any molecule which is able to connect the peptide at multiple points to form multiple sulfone bonds to the peptide. It is not a cross-linker, in that it does not normally link two peptides; instead, it provides two or more attachment points for a single peptide. Preferably, the molecular scaffold molecule comprises at least three attachment points for the peptide, referred to as scaffold reactive groups. These groups are capable of reacting with reactive groups on the peptide to form thioether linkages, which are then oxidized to the sulfone linkages. Thus, the molecular scaffold represents the scaffold moiety up to but not including the sulfone -SO₂- group in the linkages in the conjugates of the invention. The scaffold molecule has the structure of the scaffold, but with reactive groups at the locations of the sulfone linkages in the conjugate of the invention. The scaffold and/or the scaffold molecule suitably has a molecular weight of less than about 500 daltons, for example less than about 300 daltons. Preferred structures for scaffolds and scaffold molecules are as follows.

Suitably, the scaffold comprises, consists essentially of, or consists of a (hetero)aromatic or (hetero)alicyclic moiety.

As used herein, "(hetero)aryl" is meant to include aromatic rings, for example, aromatic rings having from 4 to 12 members, such as phenyl rings. These aromatic rings can optionally contain one or more heteroatoms (e.g., one or more of N, O, S, and P), such as thienyl rings, pyridyl rings, and furanyl rings. The aromatic rings can be optionally substituted. "(hetero)aryl" is also meant to include aromatic rings to which are fused one or more other aryl rings or non-aryl rings. For example, naphthyl groups, indole groups, thienothienyl groups, dithienothienyl, and 5,6,7,8-tetrahydro-2-naphthyl groups (each of which can be optionally substituted) are aryl groups for the purposes of the present application. As indicated above, the aryl rings can be optionally substituted. Suitable substituents include alkyl groups (which can optionally be substituted), other aryl groups (which may themselves be substituted), heterocyclic rings (saturated or unsaturated), alkoxy groups (which is meant to include aryloxy groups (e.g., phenoxy groups)), hydroxy groups, aldehyde groups, nitro groups, amine groups (e.g., unsubstituted, or mono- or di-substituted with aryl or alkyl groups), carboxylic acid groups, carboxylic acid derivatives (e.g., carboxylic acid esters, amides, etc.), halogen atoms (e.g., Cl, Br, and I), and the like.

As used herein, "(hetero)alicycfic" refers to a homocyclic or heterocyclic saturated ring. The ring can be unsubstituted, or it can be substituted with one or more substituents. The substituents can be saturated or unsaturated, aromatic or nonaromatic, and examples of suitable substituents include those recited above in the discussion relating to substituents on alkyl and aryl groups. Furthermore, two or more ring substituents can combine to form another ring, so that "ring", as used herein, is meant to include fused ring systems.

Suitably, the scaffold comprises a *tris*-substituted (hetero)aromatic or (hetero)alicyclic moiety, for example a tris-methylene substituted (hetero)aromatic or (hetero)alicyclic moiety. The (hetero)aromatic or (hetero)alicyclic moiety is suitably a six-membered ring structure, preferably *tris*-substituted such that the scaffold has a 3-fold symmetry axis.
In embodiments, the scaffold is a *tris*-methylene (hetero)aryl moiety, for example a 1,3,5-*tris* methylene phenyl moiety. In these embodiments, the corresponding scaffold molecule suitably has -OH or a leaving group on the methylene carbons. In these methylene substituted (hetero)aromatic compounds, the electrons of the aromatic ring can stabilize the transition state during nucleophilic substitution. Thus, for example, benzyl halides are 100-1000 times more reactive towards nucleophilic substitution than alkyl halides that are not connected to a a (hetero)aromatic group.

In these embodiments the scaffold and scaffold molecule have the general formula:

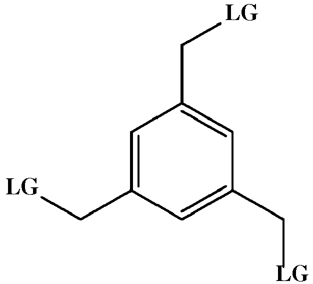

Where LG represents a leaving group as described further below for the scaffold molecule, or LG represents the sulfone -SO₂- linkage to the peptide in the conjugates of the invention.

In embodiments, the group LG above may be a bromine atom, in which case the scaffold molecule is 1,3,5-Tris(bromomethyl)benzene (TBMB). Another suitable molecular scaffold molecule is 2,4,6-Tris(bromomethyl)mesitylene. It is similar to 1,3,5-Tris(bromomethyl) benzene but contains additionally three methyl groups attached to the benzene ring. In the case of this scaffold, the additional methyl groups may form further contacts with the peptide and hence add additional structural constraint. Thus, a different diversity range is achieved than with 1,3,5-Tris(bromomethyl)benzene.

In other embodiments, the scaffold molecule may be a (hetero)aromatic or (hetero)alicyclic moiety substituted with two or more acryloyl groups, such as acrylamide or acrylate groups. These groups can undergo α,β-addition reactions with -SH to form thioether bonds. A typical scaffold molecule of this type is 1,3,5-triacryloyl-1,3,5-triazinane (TATA):

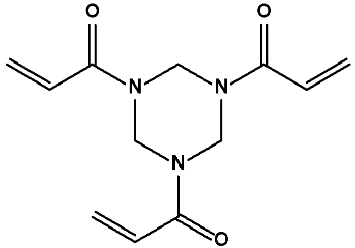

In yet other embodiments the molecular scaffold may have a tetrahedral geometry such that reaction of four functional groups of the encoded peptide with the molecular scaffold generates not more than two product isomers. Other geometries are also possible; indeed, an almost infinite number of scaffold geometries is possible, leading to greater possibilities for peptide derivative diversification.

In a second aspect, the present invention provides a method of making a compound according to the first aspect of the invention, the method comprising: providing a peptide having at least two cysteine residues; providing a scaffold molecule having at least two reactive sites for forming thioether linkages with the cysteine residues; forming said thioether linkages between the peptide and the scaffold molecule; and oxidizing the thioether linkages to sulfone linkages.

The peptide and the scaffold molecule are suitably as defined above in relation to the first aspect of the invention.

The step of reacting the peptide and the scaffold molecule to form the cyclic or bicyclic structure having thioether linkages may be performed as described in WO2004/077062 or WO2006/078161. The step of oxidizing the thioether linkages to sulfone linkages may be performed by suitable well-known oxidizing agents, for example by aqueous magnesium monoperoxy phthalate in aqueous solution (2mg/ml) at room temperature for 60 minutes.

In a further aspect, the present invention provides a compound comprising at least one looped peptide structure attached via at least two sulfone linkages formed through cysteine residues of the peptide to a scaffold, wherein the Kd of the compound towards a predetermined target molecule is no more than about 20-fold the Kd of the corresponding compound having thioether linkages in place of the sulfone linkages.

In alternative aspects of the invention Kd may be replaced by Ki or by IC50. Suitably, the Kd of the compound towards a predetermined target molecule is no more than about 20 times the Kd of the corresponding compound having thioether linkages in place of the sulfone linkages. Suitably, the claimed peptide compound having sulfone linkages has Kd and/or Ki with the predetermined target less than about 50nM.

Suitably, the Kd, Ki and/or IC50 of the compound towards a predetermined target molecule is no more than about 10x the Kd of the corresponding compound having thioether linkages in place of the sulfone linkages.

The peptide element of the peptide conjugate of the invention according to this aspect comprises two or more reactive groups, preferably three or more reactive groups, which are responsible for binding to the scaffold; and loop sequences between the reactive groups. Diversity can be obtained, as in the prior art, by varying the sequence of the loops. The reactive group for conjugating the peptide to the molecular scaffold is, in one embodiment, - -SH, in particular cysteine -SH.

A particular advantage of the peptide conjugates of this and other aspects of the invention is that they are smaller binding agents of the prior art. Typically, a compound of the present invention has a molecular weight of less than about 5000 Dalton; preferably less than about 4000 Dalton; and preferably less than about 3000 Dalton. It will be understood that a derivative constructed with multiple peptide derivatives may have a higher molecular weight outside these ranges. Moreover, peptide derivatives bound to molecules such as HSA will have a much higher molecular weight.

The small size of the compounds of the invention results from the use of small molecular scaffolds, typically up to about 500 Dalton in mass. The peptide itself is preferably less than about 27 amino acids in length, as measured between the N-terminal and C-terminal attachment points which attach it to the molecular scaffold. Further peptides may, of course, be present or be attached outside of the attachment points, lengthening the peptide structure. Each loop of the peptide is preferably between 0 and 9 amino acids in length, measured between adjacent attachment points. Advantageously, the loops in any peptide derivative are independently 3, 4, 5, 6, 7, 8 or 9 amino acids in length.

Several conjugated peptides may be incorporated together into the same molecule according to the present invention. For example two such peptide conjugates of the same specificity can be linked together via the molecular scaffold, increasing the avidity of the derivative for its targets. Alternatively, in another embodiment a plurality of peptide conjugates are combined to form a multimer. For example, two different peptide conjugates are combined to create a multispecific molecule. Alternatively, three or more peptide conjugates, which may be the same or different, can be combined to form multispecific derivatives. In one embodiment multivalent complexes may be constructed by linking together the molecular scaffolds, which may be the same or different.

The peptide comprises a molecular scaffold binding segment. This is the region to which the molecular scaffold is attached. Suitably the commentary regarding reactive groups on the peptide is applied to this binding segment. Suitably the molecular scaffold binding segment of the target peptide comprises 1 to 27 amino acid residues, suitably 5 to 20 amino acid residues. Suitably the molecular scaffold binding segment of the target peptide comprises fewer than 10 amino acids. This has the advantage of imposing further conformational constraint onto the peptide segment when it is attached to the molecular scaffold.

The peptide suitably comprises the sequence (X)ₗY(X)ₘY(X)ₙY(X)ₒ, wherein Y represents an amino acid with a reactive group for forming the sulfone linkage (such as cysteine), X represents a random amino acid, m and n are numbers between 1 and 20 defining the length of intervening peptide segments and l and o are numbers between 0 and 20 defining the length of the flanking peptide segments.

In embodiments, the peptide suitably comprises the sequence XS(X)₆S(X)₆SX, wherein X stands for a random natural amino acid and S stands for cysteine. In other embodiments, the peptide derivative of the invention may comprise a peptide with the sequence S(X)₆S(X)₆S

The target is a molecule or part thereof to which the peptide derivatives bind. Typically, the predetermined target will be analogous to an epitope. One skilled in the art will appreciate that the choice of target molecule is large and varied. They may be for instance human or animal proteins, cytokines, cytokine receptors, enzymes co-factors for enzymes or DNA binding proteins. Suitable cytokines and growth factors include but are not limited to: ApoE, Apo-SAA, BDNF, Cardiotrophin-1, EGF, EGF receptor, ENA78, Eotaxin, Eotaxin-2, Exodus-2, FGF-acidic, FGF-basic, fibroblast growth factor-10 (30). FLT3 derivative, Fractalkine (CX3C), GDNF, G-CSF, GM-CSF, GF- 1, insulin, IFNγ, IGF-I, IGF-II, IL-la, IL-1 (3, IL-2, IL-3, IL-4, IL-5, IL-6, IL-7, IL-8 (72 a.a.), IL-8 (77 a.a.), IL-9, IL-10, IL-11, IL-12, IL-13, IL-15, IL-16, IL-17, IL-17a, IL-17c,IL-17d, IL-17e, IL-17f, IL-18 (IGIF), IL-21, IL-22, IL-23, IL-31, IL-32, IL-33, IL-34, Inhibin α, Inhibin β, IP-10, keratinocyte growth factor-2 (KGF-2), KGF, Leptin, LIF, Lymphotactin, Mullerian inhibitory substance, monocyte colony inhibitory factor, monocyte attractant protein (30 ibid), M-CSF, MDC (67 a. a.), MDC (69 a. a.), MCP-1 (MCAF), MCP-2, MCP-3, MCP-4, MDC (67 a. a.), MDC (69 a. a.), MIG, MIP-la, MIP-1p, MIP-3a, MIP3 (3, MIP-4, myeloid progenitor inhibitor factor-1 (MPIF-1), NAP-2, Neurturin, Nerve growth factor, P-NGF, NT-3, NT-4, Oncostatin M, PDGF-AA, PDGF-AB, PDGF-BB, PF-4, RANTES, SDFla, SDFlp, SCF, SCGF, stem cell factor (SCF), TARC, TGF-α, TGF-β, TGF- 2, TGF- 3, tumour necrosis factor (TNF), TNF-α, TNF-β, TNF receptor I, TNF receptor II, TNIL-1, TPO, VEGF, VEGF receptor 1, VEGF receptor 2, VEGF receptor 3, GCP-2, GRO/MGSA, GRO-β, GRO-γ, HCC1,1-309, HER 1, HER 2, HER 3 and HER 4. Cytokine receptors include receptors for the foregoing cytokines. Chemokine targets include CC chemokine derivatives CCL21/6Ckine, CCL12/MCP-5, CCL6/C10, CCL22/MDC, CCL14/HCC-1/HCC-3, CCL3L1/MIP-1 alpha Isoform LD78 beta, CCL23/Ck beta 8-1, CCL3/MIP-1 alpha, CCL28, CCL4L1/LAG-1, CCL27/CTACK, CCL4/MIP-1 beta, CCL24/Eotaxin-2/MPIF-2, CCL15/MIP-1 delta, CCL26-like/Eotaxin-3-like, CCL9/10/MIP-1 gamma, CCL26/Eotaxin-3, CCL19/MIP-3 beta, CCL11/Eotaxin,CCL20/MIP-3 alpha, CCL14a/HCC-1, CCL23/MPIF-1, CCL14b/HCC-3, CCL18/PARC, CCL16/HCC-4, CCL5/RANTES, CCL1/I-309/TCA-3, TAFA1/FAM19A1, MCK-2, TAFA5/FAM19A5, CCL2/JE/MCP-1, TAFA3/FAM19A3, CCL8/MCP-2, TAFA4/FAM19A4, CCL7/MCP-3/MARC, CCL17/TARC, CCL13/MCP-4 and CCL25/TECK; chemokine receptors include CCR1, CCR7, CCR2, CCR8, CCR3, CCR9, CCR4, CCR10, CCR5, CCRL2/LCCR/CRAM-A/B and CCR6; CXC chemokine derivatives include CXCL13/BLC/BCA-1, CXCL10/IP-10/CRG-2, CXCL14/BRAK, LIX, CXCL16, CXCL15/Lungkine, CXCL5/ENA-78, CXCL9/MIG, CXCL6/GCP-2, CXCL7/NAP-2, CXCL1/2/3/GRO, CXCL4/PF4, CXCL1/GRO alpha/KC/CINC-1, CXCL12/SDF-1 alpha, CXCL2/GRO beta/MIP-2/CINC-3, CXCL12/SDF-1 beta, CXCL3/GRO gamma/CINC-2/DCIP-1, CXCL12/SDF-1, CXCL11/I-TAC, CXCL7/Thymus Chemokine-1 and CXCL8/IL-8; CXC chemokine receptors include CXCR3, CXCR7/RDC-1, CXCR4, CXCR1/IL-8 RA, CXCR5, CXCR2/IL-8 RB and CXCR6; TNF Superfamily derivatives include 4-1BB Derivative/TNFSF9, LIGHT/TNFSF14, APRIL/TNFSF13, Lymphotoxin, BAFF/BLyS/TNFSF13B, Lymphotoxin beta/TNFSF3, CD27 Derivative/TNFSF7, OX40 Derivative/TNFSF4, CD30 Derivative/TNFSF8, TL1A/TNFSF15, CD40 Derivative/TNFSF5, TNF-alpha/TNFSF1A, EDA (pan), TNF-beta/TNFSF1B, EDA-A1/Ectodysplasin A1, TRAIL/TNFSF10, EDA-A2, TRANCE/TNFSF11, Fas Derivative/TNFSF6, TWEAK/TNFSF12 and GITR Derivative/TNFSF18; TNF Superfamily receptors include 4-1BB/TNFRSF9/CD137, NGF R/TNFRSF16, BAFF R/TNFRSF13C, Osteoprotegerin/TNFRSF11B, BCMA/TNFRSF17, OX40/TNFRSF4, CD27/TNFRSF7, RANK/TNFRSF11A, CD30/TNFRSF8, RELT/TNFRSF19L, CD40/TNFRSF5, TACI/TNFRSF13B, DcR3/TNFRSF6B, TNFRH3/TNFRSF26, DcTRAIL R1/TNFRSF23, TNF RI/TNFRSF1A, DcTRAIL R2/TNFRSF22, TNF RII/TNFRSF1B, DR3/TNFRSF25, TRAIL R1/TNFRSF10A, DR6/TNFRSF21, TRAIL R2/TNFRSF10B, EDAR, TRAIL R3/TNFRSF10C, Fas/TNFRSF6/CD95, TRAIL R4/TNFRSF10D, GITR/TNFRSF18, TROY/TNFRSF19, HVEM/TNFRSF14, TWEAK R/TNFRSF12, Lymphotoxin beta R/TNFRSF3 and XEDAR; Toll-Like Receptors including TLR-1, TLR-2, TLR-3, TLR-4, TLR-5, TLR-6, TLR-7, TLR-8 and TLR-9; enzymes, including Cathepsin A, Cathepsin B, Cathepsin C, Cathepsin D, Cathepsin E, Cathepsin F, MMP 1, MMP2, MMP 3, MMP 7, MMP 8, MMP 9, MMP 10, MMP 11, MMP 12, MMP 13, MMP 14, MMP 15, MMP 16, MMP 17, MMP 19, MMP 20, MMP 21, MMP 23A, MMP 23B, MMP 26, MMP 27, MMP 28, urokinase, kallikreins, including KLK1, KLK2, KLK3, KLK4, KLK5, KLK6, KLK7, KLK8, KLK9, KLK10, KLK11, KLK12, KLK13, KLK14 and KLK15; components of the complement system; Intracellular signalling molecules and transcription factors; p53; and MDM2. Targets may also be large plasma proteins, such as serum albumins, as set forth below. It will be appreciated that this list is by no means exhaustive.

In this and other aspects of the invention, additional binding or functional activities may be attached to the N or C terminus of the peptide covalently linked to a molecular scaffold. The functional group is, for example, selected from the group consisting of: a group capable of binding to a molecule which extends the half-life of the peptide derivative *in vivo*, and a molecule which extends the half-life of the peptide derivative *in vivo*. Such a molecule can be, for instance, HSA or a cell matrix protein, and the group capable of binding to a molecule which extends the half-life of the peptide derivative in vivo is an antibody or antibody fragment specific for HSA or a cell matrix protein.

In one embodiment, the functional group is a binding molecule, selected from the group consisting of a second peptide derivative comprising a peptide covalently linked to a molecular scaffold, and an antibody or antibody fragment. 2, 3, 4, 5 or more peptide derivatives may be joined together. The specificities of any two or more of these derivatives may be the same or different; if they are the same, a multivalent binding structure will be formed, which has increased avidity for the target compared to univalent binding molecules. The molecular scaffolds, moreover, may be the same or different, and may subtend the same or different numbers of loops.

The functional group can moreover be an effector group, for example an antibody Fc region.

Attachments to the N or C terminus may be made prior to binding of the peptide to a molecular scaffold, or afterwards. Thus, the peptide may be produced (synthetically, or by expression of nucleic acid) with an N or C terminal peptide group already in place. Preferably, however, the addition to the N or C terminus takes place after the peptide has been combined with the molecular backbone to form a conjugate. For example, Fluorenylmethyloxycarbonyl chloride can be used to introduce the Fmoc protective group at the N-terminus of the peptide. Fmoc binds to serum albumins including HSA with high affinity, and Fmoc-Trp or Fmoc-Lys bind with an increased affinity. The peptide can be synthesised with the Fmoc protecting group left on, and then coupled with the scaffold through the cysteines. An alternative is the palmitoyl moiety which also binds HSA and has, for example been used in Liraglutide to extend the half-life of this GLP-1 analogue.

The Fmoc group confers human serum albumin binding function to the bicyclic peptide. Alternatively, a conjugate of the peptide with the scaffold can be made, and then modified at the N-terminus, for example with the amine- and sulfhydryl-reactive linker N-e-maleimidocaproyloxy)succinimide ester (EMCS). Via this linker the peptide conjugate can be linked to other peptides, for example an antibody Fc fragment.

The binding function may be another peptide bound to a molecular scaffold, creating a multimer; another binding protein, including an antibody or antibody fragment; or any other desired entity, including serum albumin or an effector group, such as an antibody Fc region.

Additional binding or functional activities can moreover be bound directly to the molecular scaffold.

In embodiments, the scaffold may further comprise a reactive group to which the additional activities can be bound. Preferably, this group is orthogonal with respect to the other reactive groups on the molecular scaffold, to avoid interaction with the peptide. In one embodiment, the reactive group may be protected, and deprotected when necessary to conjugate the additional activities.

According to a further aspect of the invention, there is provided a drug conjugate comprising a peptide ligand as defined herein conjugated to one or more effector and/or functional groups.

Effector and/or functional groups can be attached, for example, to the N and/or C termini of the polypeptide, to an amino acid within the polypeptide, or to the molecular scaffold.

Appropriate effector groups include antibodies and parts or fragments thereof. For instance, an effector group can include an antibody light chain constant region (CL), an antibody CH1 heavy chain domain, an antibody CH2 heavy chain domain, an antibody CH3 heavy chain domain, or any combination thereof, in addition to the one or more constant region domains. An effector group may also comprise a hinge region of an antibody (such a region normally being found between the CH1 and CH2 domains of an IgG molecule).

In a further embodiment of this aspect of the invention, an effector group according to the present invention is an Fc region of an IgG molecule. Advantageously, a peptide ligand-effector group according to the present invention comprises or consists of a peptide ligand Fc fusion having a tβ half-life of a day or more, two days or more, 3 days or more, 4 days or more, 5 days or more, 6 days or more or 7 days or more. Most advantageously, the peptide ligand according to the present invention comprises or consists of a peptide ligand Fc fusion having a tβ half-life of a day or more.

Functional groups include, in general, binding groups, drugs, reactive groups for the attachment of other entities, functional groups which aid uptake of the macrocyclic peptides into cells, and the like.

The ability of peptides to penetrate into cells will allow peptides against intracellular targets to be effective. Targets that can be accessed by peptides with the ability to penetrate into cells include transcription factors, intracellular signalling molecules such as tyrosine kinases and molecules involved in the apoptotic pathway. Functional groups which enable the penetration of cells include peptides or chemical groups which have been added either to the peptide or the molecular scaffold. Peptides such as those derived from such as VP22, HIV-Tat, a homeobox protein of Drosophila (Antennapedia), e.g. as described in Chen and Harrison, Biochemical Society Transactions (2007) Volume 35, part 4, p821; Gupta et al. in Advanced Drug Discovery Reviews (2004) Volume 57 9637. Examples of short peptides which have been shown to be efficient at translocation through plasma membranes include the 16 amino acid penetratin peptide from Drosophila Antennapedia protein (Derossi et al (1994) J Biol. Chem. Volume 269 p10444), the 18 amino acid 'model amphipathic peptide' (Oehlke et al (1998) Biochim Biophys Acts Volume 1414 p127) and arginine rich regions of the HIV TAT protein. Non peptidic approaches include the use of small molecule mimics or SMOCs that can be easily attached to biomolecules (Okuyama et al (2007) Nature Methods Volume 4 p153). Other chemical strategies to add guanidinium groups to molecules also enhance cell penetration (Elson-Scwab et al (2007) J Biol Chem Volume 282 p13585). Small molecular weight molecules such as steroids may be added to the molecular scaffold to enhance uptake into cells.

One class of functional groups which may be attached to peptide ligands includes antibodies and binding fragments thereof, such as Fab, Fv or single domain fragments. In particular, antibodies which bind to proteins capable of increasing the half-life of the peptide ligand *in vivo* may be used.

RGD peptides, which bind to integrins which are present on many cells, may also be incorporated.

In one embodiment, a peptide ligand-effector group according to the invention has a tβ half-life selected from the group consisting of: 12 hours or more, 24 hours or more, 2 days or more, 3 days or more, 4 days or more, 5 days or more, 6 days or more, 7 days or more, 8 days or more, 9 days or more, 10 days or more, 11 days or more, 12 days or more, 13 days or more, 14 days or more, 15 days or more or 20 days or more. Advantageously a peptide ligand-effector group or composition according to the invention will have a tβ half life in the range 12 to 60 hours. In a further embodiment, it will have a tβ half-life of a day or more. In a further embodiment still, it will be in the range 12 to 26 hours.

In one particular embodiment of the invention, the functional group is selected from a metal chelator, which is suitable for complexing metal radioisotopes of medicinal relevance. Such effectors, when complexed with said radioisotopes, can present useful agents for cancer therapy. Suitable examples include DOTA, NOTA, EDTA, DTPA, HEHA, SarAr and others (Targeted Radionuclide therapy, Tod Speer, Wolters/Kluver Lippincott Williams & Wilkins, 2011).

Possible effector groups also include enzymes, for instance such as carboxypeptidase G2 for use in enzyme/prodrug therapy, where the peptide ligand replaces antibodies in ADEPT.

In one particular embodiment of the invention, the functional group is selected from a drug, such as a cytotoxic agent for cancer therapy. Suitable examples include: alkylating agents such as cisplatin and carboplatin, as well as oxaliplatin, mechlorethamine, cyclophosphamide, chlorambucil, ifosfamide; Anti-metabolites including purine analogs azathioprine and mercaptopurine or pyrimidine analogs; plant alkaloids and terpenoids including vinca alkaloids such as Vincristine, Vinblastine, Vinorelbine and Vindesine; Podophyllotoxin and its derivatives etoposide and teniposide; Taxanes, including paclitaxel, originally known as Taxol; topoisomerase inhibitors including camptothecins: irinotecan and topotecan, and type II inhibitors including amsacrine, etoposide, etoposide phosphate, and teniposide. Further agents can include antitumour antibiotics which include the immunosuppressant dactinomycin (which is used in kidney transplantations), doxorubicin, epirubicin, bleomycin, calicheamycins, and others.

In one further particular embodiment of the invention, the cytotoxic agent is selected from maytansinoids (such as DM1) or monomethyl auristatins (such as MMAE). Further details of such conjugated bicyclic peptides are given in the aforementioned Copending PCT International Patent Application of the present applicant with title "Bicyclic Peptide Ligands Specific for MT1-MMP" filed October 2015 under attorney reference BIC-C-P1803PCT, the entire disclosure of which is incorporated herein by reference.

In one embodiment, the cytotoxic agent is linked to the bicyclic peptide by a cleavable bond, such as a disulphide bond or a protease sensitive bond. In a further embodiment, the groups adjacent to the disulphide bond are modified to control the hindrance of the disulphide bond, and by this the rate of cleavage and concomitant release of cytotoxic agent.

Published work established the potential for modifying the susceptibility of the disulphide bond to reduction by introducing steric hindrance on either side of the disulphide bond (Kellogg et al (2011) Bioconjugate Chemistry, 22, 717). A greater degree of steric hindrance reduces the rate of reduction by intracellular glutathione and also extracellular (systemic) reducing agents, consequentially reducing the ease by which toxin is released, both inside and outside the cell. Thus, selection of the optimum in disulphide stability in the circulation (which minimises undesirable side effects of the toxin) versus efficient release in the intracellular milieu (which maximises the therapeutic effect) can be achieved by careful selection of the degree of hindrance on either side of the disulphide bond.

The hindrance on either side of the disulphide bond is modulated through introducing one or more methyl groups on either the targeting entity (here, the bicyclic peptide) or toxin side of the molecular construct.

Peptide derivatives according to the present invention may be employed in *in vivo* therapeutic and prophylactic applications, *in vitro* and *in vivo* diagnostic applications, in vitro assay and reagent applications, and the like.

In general, the use of a peptide derivative can replace that of an antibody. Derivatives selected according to the invention are of use diagnostically in Western analysis and in situ protein detection by standard immunohistochemical procedures; for use in these applications, the derivatives of a selected repertoire may be labelled in accordance with techniques known in the art. In addition, such peptide derivatives may be used preparatively in affinity chromatography procedures, when complexed to a chromatographic support, such as a resin. All such techniques are well known to one of skill in the art. Peptide derivatives according to the present invention possess binding capabilities similar to those of antibodies, and may replace antibodies in such assays.

Diagnostic uses include any uses which to which antibodies are normally put, including test-strip assays, laboratory assays and immunodiagnostic assays.

Therapeutic and prophylactic uses of peptide derivatives prepared according to the invention involve the administration of derivatives selected according to the invention to a recipient mammal, such as a human. Substantially pure peptide derivatives of at least 90 to 95% homogeneity are preferred for administration to a mammal, and 98 to 99% or more homogeneity is most preferred for pharmaceutical uses, especially when the mammal is a human. Once purified, partially or to homogeneity as desired, the selected peptides may be used diagnostically or therapeutically (including extracorporeally) or in developing and performing assay procedures, immunofluorescent stainings and the like (Lefkovite and Pernis, (1979 and 1981) Immunological Methods, Volumes I and II, Academic Press, NY). The peptide derivatives of the present invention will typically find use in preventing, suppressing or treating inflammatory states, allergic hypersensitivity, cancer, bacterial or viral infection, and autoimmune disorders (which include, but are not limited to, Type I diabetes, multiple sclerosis, rheumatoid arthritis, systemic lupus erythematosus, Crohn's disease and myasthenia gravis).

In the instant application, the term "prevention" involves administration of the protective composition prior to the induction of the disease. "Suppression" refers to administration of the composition after an inductive event, but prior to the clinical appearance of the disease. "Treatment" involves administration of the protective composition after disease symptoms become manifest.

Animal model systems which can be used to screen the effectiveness of the peptide derivatives in protecting against or treating the disease are available.

Methods for the testing of systemic lupus erythematosus (SLE) in susceptible mice are known in the art (Knight et al. (1978) J Exp. Med., 147: 1653; Reinersten et al. (1978) New Eng. J : Med., 299: 515). Myasthenia Gravis (MG) is tested in SJL/J female mice by inducing the disease with soluble AchR protein from another species (Lindstrom et al. (1988) Adv. Inzn7unol., 42: 233). Arthritis is induced in a susceptible strain of mice by injection of Type II collagen (Stuart et al. (1984) Ann. Rev. Immunol., 42: 233). A model by which adjuvant arthritis is induced in susceptible rats by injection of mycobacterial heat shock protein has been described (Van Eden et al. (1988) Nature, 331: 171). Thyroiditis is induced in mice by administration of thyroglobulin as described (Maron et al. (1980) J. Exp. Med., 152: 1115). Insulin dependent diabetes mellitus (IDDM) occurs naturally or can be induced in certain strains of mice such as those described by Kanasawa et al. (1984) Diabetologia, 27: 113. EAE in mouse and rat serves as a model for MS in human. In this model, the demyelinating disease is induced by administration of myelin basic protein (see Paterson (1986) Textbook of Immunopathology, Mischer et al., eds. , Grune and Stratton, New York, pp. 179-213; McFarlin et al. (1973) Science, 179: 478: and Satoh et al. (1987) J ; Immunol., 138: 179).

Generally, the present peptide derivatives will be utilised in purified form together with pharmacologically appropriate carriers. Typically, these carriers include aqueous or alcoholic/aqueous solutions, emulsions or suspensions, any including saline and/or buffered media. Parenteral vehicles include sodium chloride solution, Ringer's dextrose, dextrose and sodium chloride and lactated Ringer's. Suitable physiologically-acceptable adjuvants, if necessary to keep a peptide complex in suspension, may be chosen from thickeners such as carboxymethylcellulose, polyvinylpyrrolidone, gelatin and alginates.

Intravenous vehicles include fluid and nutrient replenishers and electrolyte replenishers, such as those based on Ringer's dextrose. Preservatives and other additives, such as antimicrobials, antioxidants, chelating agents and inert gases, may also be present (Mack (1982) Remington's Pharmaceutical Sciences, 16th Edition).

The peptide derivatives of the present invention may be used as separately administered compositions or in conjunction with other agents. These can include antibodies, antibody fragments and various immunotherapeutic drugs, such as cyclosporine, methotrexate, adriamycin or cisplatinum, and immunotoxins. Pharmaceutical compositions can include "cocktails" of various cytotoxic or other agents in conjunction with the selected antibodies, receptors or binding proteins thereof of the present invention, or even combinations of selected peptides according to the present invention having different specificities, such as peptides selected using different target derivatives, whether or not they are pooled prior to administration.

The route of administration of pharmaceutical compositions according to the invention may be any of those commonly known to those of ordinary skill in the art. For therapy, including without limitation immunotherapy, the selected antibodies, receptors or binding proteins thereof of the invention can be administered to any patient in accordance with standard techniques. The administration can be by any appropriate mode, including parenterally, intravenously, intramuscularly, intraperitoneally, transdermally, via the pulmonary route, or also, appropriately, by direct infusion with a catheter. The dosage and frequency of administration will depend on the age, sex and condition of the patient, concurrent administration of other drugs, counter-indications and other parameters to be taken into account by the clinician.

The peptide derivatives of this invention can be lyophilised for storage and reconstituted in a suitable carrier prior to use. This technique has been shown to be effective and art-known lyophilisation and reconstitution techniques can be employed. It will be appreciated by those skilled in the art that lyophilisation and reconstitution can lead to varying degrees of activity loss and that use levels may have to be adjusted upward to compensate.

The compositions containing the present peptide derivatives or a cocktail thereof can be administered for prophylactic and/or therapeutic treatments. In certain therapeutic applications, an adequate amount to accomplish at least partial inhibition, suppression, modulation, killing, or some other measurable parameter, of a population of selected cells is defined as a "therapeutically-effective dose". Amounts needed to achieve this dosage will depend upon the severity of the disease and the general state of the patient's own immune system, but generally range from 0.005 to 5.0 mg of selected peptide derivative per kilogram of body weight, with doses of 0.05 to 2.0 mg/kg/dose being more commonly used. For prophylactic applications, compositions containing the present peptide derivatives or cocktails thereof may also be administered in similar or slightly lower dosages.

A composition containing a peptide derivative according to the present invention may be utilised in prophylactic and therapeutic settings to aid in the alteration, inactivation, killing or removal of a select target cell population in a mammal. In addition, the selected repertoires of peptides described herein may be used extracorporeally or in vitro selectively to kill, deplete or otherwise effectively remove a target cell population from a heterogeneous collection of cells. Blood from a mammal may be combined extracorporeally with the selected peptide derivatives whereby the undesired cells are killed or otherwise removed from the blood for return to the mammal in accordance with standard techniques.

The invention is further described with reference to the following examples.

### Example 1

### Synthesis of tris-sulfone Bicyclic Peptides Targeting Human MT1-MMP

The Bicyclic Peptide chosen for thioether to sulfone substitution was 17-69-07-N241, which has peptide sequence:
H-(b-Ala)-Sar₁₀-Ala-Cys-(D-Ala)-Asn-Glu-(1Nal)-(D-Ala)-Cys-Glu-Asp-Phe-Tyr-Asp-(tBuGly)-Cys-NH2
wherein Sar is sarcosine, where D-Ala is D-Alanine, where 1NAl is 1-naphthyl-L-alanine, where tBuGly is α-t-butylglycine and where b-Ala is β-Alanine. The sequence was cyclised and purified using 1,3,5 tris-bromomethylbenzene as previously disclosed. The resulting thioether-linked bicyclic compound has a mass of 2657.9 Da.

The compound was oxidised under mildly acidic conditions, in aqueous, solvent-free solution, at room temperature: 100 mg 17-69-07-N241 was dissolved in 50 mM ammonium acetate pH 5.5 in water such that the Bicyclic Peptide concentration was ∼10.4 mM in a volume of 3.6 mL. 306 mg of magnesium monoperoxyphthalate (13 equivalents) was directly added to the mixture, and stirred at room temperature. Samples were withdrawn periodically and analysed using MALDI-ToF mass spectrometry (due to the oxidation, the mass increase is 6 x 16 = 96 Da), and the reaction was judged complete within 30 minutes, with no lower oxidised species visible.

Following completion of the reaction, the reaction was quenched by the addition of 200 mM cysteine (150 µL), diluted and loaded onto a Phenomenex C18 column and purified by a standard reverse phase gradient. Eligible fractions were pooled and lyophilised, and recovered yield was weighed at 60 mg. The purified tris-sulfone compound (termed 17-69-07-N338) was analysed comparatively to the parent compound 17-69-07-N241 by LC-MS, and found to have a retention time of 4.53 min compared to 4.99 min for the 17-69-07-N241 parent compound. This demonstrated that the tris-sulfone compound 17-69-07-N338 was purified efficiently and free of parent material.

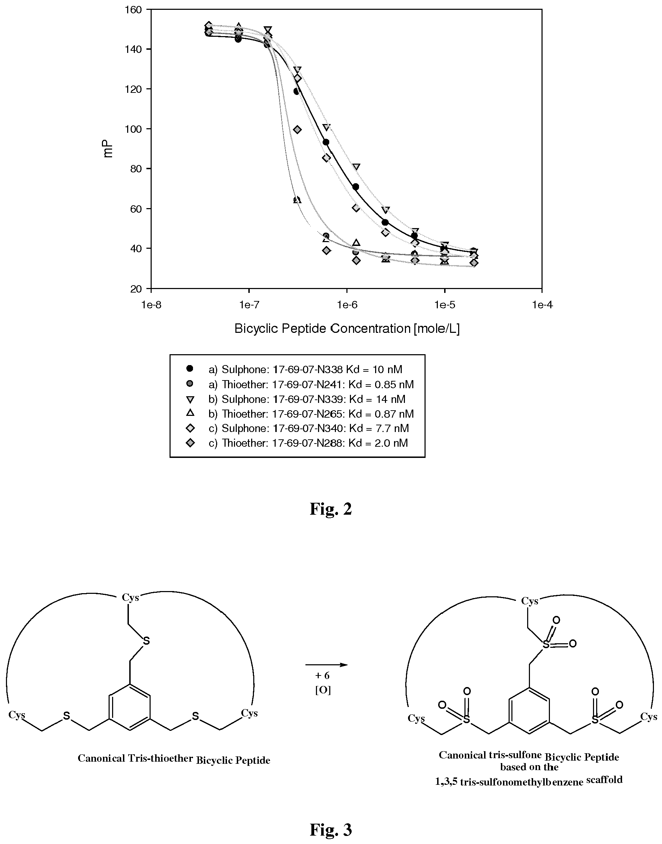

Subsequently, the affinity to the MT1-MMP target was measured by fluorescence polarisation competition experiments for 17-69-07-N241, and its sulfone versions (Figure 2).

The data shows a small reduction of affinity to ∼ 10 nM for 17-69-07-N338. This affinity is sufficiently potent for therapeutic applications. Generation of the tris-sulfone of 17-69-07-N241 therefore has generated a molecule lacking the oxidation sensitivity with mostly retained biological activity.

### Example 2

A further bicyclic peptide, 17-69-07-N265 was prepared and oxidized by the method described in Example 1. This has peptide sequence identical to 17-69-07-N241, except for N-terminal acetylation:
Acetyl-(b-Ala)-Sar₁₀-Ala-Cys-(D-Ala)-Asn-Glu-(1Nal)-(D-Ala)-Cys-Glu-Asp-Phe-Tyr-Asp-(tBuGly)-Cys-NH2

The sulfone version of this Bicyclic Peptide could be generated readily in a manner described above. The affinity of the molecules to MT1-MMP were determined as above, and the data shows a comparable small reduction of affinity between the thioether and sulfone versions of the peptide (Figure 2).

### Example 3

A further bicyclic peptide, 17-69-07-N288 was prepared and oxidized by the method described in Example 1. This has peptide sequence identical to 17-69-07-N241, except the sequence lacks the H-(b-Ala)-Sar₁₀- moiety, and has a 4-bromophenylalanine in place of tyrosine:
H-Ala-Cys-(D-Ala)-Asn-Glu-(1Nal)-(D-Ala)-Cys-Glu-Asp-Phe-(4BrPhe)-Asp-(tBuGly)-Cys-NH2

The sulfone version of this Bicyclic Peptide could be generated readily in a manner described above. The affinity of the molecules to MT1-MMP were determined as above, and the data shows a comparable small reduction of affinity between the thioether and sulfone versions of the peptide (Figure 2).

All publications mentioned in the above specification are herein incorporated by reference. Various modifications and variations of the described aspects and embodiments of the present invention will be apparent to those skilled in the art without departing from the scope of the present invention. Although the present invention has been described in connection with specific preferred embodiments, it should be understood that the invention as claimed should not be unduly limited to such specific embodiments. Indeed, various modifications of the described modes for carrying out the invention which are apparent to those skilled in the art are intended to be within the scope of the following claims.

## Claims

1. A compound comprising at least one looped peptide structure attached via at least two sulfone linkages formed through cysteine residues of the peptide to a scaffold, wherein the looped peptide comprises an amino acid sequence of formula (I):
- Cᵢ-X-U/O-X-X-G-Cᵢᵢ-E-D-F-Y-X-X-Cᵢᵢᵢ- (SEQ ID NO: 1) (I)
or a modified derivative, or pharmaceutically acceptable salt, thereof;
wherein:
Cᵢ, Cᵢᵢ and Cᵢᵢᵢ represent first, second and third cysteine residues, respectively;
X represents any amino acid residue;
U represents a polar, uncharged amino acid residue selected from N, C, Q, M, S and T; and
O represents a non-polar aliphatic amino acid residue selected from G, A, I, L, P and V.

2. The compound as defined in claim 1, wherein the X at position 1 is selected from any one of the following amino acids: Y, M, F or V, such as Y, M or F, in particular, Y or M, more particularly Y.

3. The compound as defined in claim 1 or claim 2, wherein the U/O at position 2 is selected from a U, such as an N, or an O, such as a G.

4. The compound as defined in any one of claims 1 to 3, wherein the X at position 3 is selected from U or Z, wherein U represents a polar, uncharged amino acid residue selected from N, C, Q, M, S and T and Z represents a polar, negatively charged amino acid residue selected from D or E, in particular the U at position 3 is selected from Q or the Z at position 3 is selected from E.

5. The compound as defined in any one of claims 1 to 4, wherein the X at position 4 is selected from J, wherein J represents a non-polar aromatic amino acid residue selected from F, W and Y.

6. The compound as defined in any one of claims 1 to 5, wherein the X at position 10 is selected from Z, wherein Z represents a polar, negatively charged amino acid residue selected from D or E, such as D.

7. The compound as defined in any one of claims 1 to 6, wherein the X at position 11 is selected from O, wherein O represents a non-polar aliphatic amino acid residue selected from G, A, I, L, P and V, such as I.

8. The compound as defined in any one of claims 1 to 7, wherein the compound of formula (I) is a compound of formula (Ia):
-Cᵢ-Y/M/F/V-U/O-U/Z-J-G-Cᵢᵢ-E-D-F-Y-Z-O-Cᵢᵢᵢ- (SEQ ID NO: 6) (Ia)
wherein U, O, J and Z are as defined hereinbefore; or a compound of formula (Ib):
-Cᵢ-Y/M/F/V-N/G-E/Q-F-G-Cᵢᵢ-E-D-F-Y-D-I-Cᵢᵢᵢ- (SEQ ID NO: 7) (Ib);
or
a compound of formula (Ic):
-Cᵢ-Y/M/F-N/G-E/Q-F-G-Cᵢᵢ-E-D-F-Y-D-I-Cᵢᵢᵢ- (SEQ ID NO: 8) (Ic);
or
a compound of formula (Id):
-Cᵢ-Y/M-N-E/Q-F-G-Cᵢᵢ-E-D-F-Y-D-I-Cᵢᵢᵢ- (SEQ ID NO: 9) (Id);
or
a compound of formula (Ie):
-Cᵢ-Y-N-E-F-G-Cᵢᵢ-E-D-F-Y-D-I-Cᵢᵢᵢ- (17-69-07) (SEQ ID NO: 2) (Ie).

9. The compound as defined in any one of claims 1 to 8, wherein the peptide of formula (I) comprises a sequence selected from:
-Cᵢ-Y-N-E-F-G-Cᵢᵢ-E-D-F-Y-D-I-Cᵢᵢᵢ- (17-69-07) (SEQ ID NO: 2);
-Cᵢ-M-N-Q-F-G-Cᵢᵢ-E-D-F-Y-D-I-Cᵢᵢᵢ- (17-69-12) (SEQ ID NO: 10);
-Cᵢ-F-G-E-F-G-Cᵢᵢ-E-D-F-Y-D-I-Cᵢᵢᵢ- (17-69-02) (SEQ ID NO: 11);
-Cᵢ-V-N-E-F-G-Cᵢᵢ-E-D-F-Y-D-I-Cᵢᵢᵢ- (17-69-03) (SEQ ID NO: 12);
-Cᵢ-F-N-E-F-G-Cᵢᵢ-E-D-F-Y-D-I-Cᵢᵢᵢ- (17-69-04) (SEQ ID NO: 13);
-Cᵢ-Y-N-E-Y-G-Cᵢᵢ-E-D-F-Y-D-I-Cᵢᵢᵢ- (17-69-07-N057) (SEQ ID NO: 14); and
-Cᵢ-Y-N-E-W-G-Cᵢᵢ-E-D-F-Y-D-I-Cᵢᵢᵢ- (17-69-44-N002) (SEQ ID NO: 15),
such as:
-Cᵢ-Y-N-E-F-G-Cᵢᵢ-E-D-F-Y-D-I-Cᵢᵢᵢ- (17-69-07) (SEQ ID NO: 2); and
-Cᵢ-M-N-Q-F-G-Cᵢᵢ-E-D-F-Y-D-I-Cᵢᵢᵢ- (17-69-12) (SEQ ID NO: 10),
in particular:
-Cᵢ-Y-N-E-F-G-Cᵢᵢ-E-D-F-Y-D-I-Cᵢᵢᵢ- (17-69-07) (SEQ ID NO: 2).

10. The compound as defined in claim 9, wherein the peptide of formula (I) has sequence:
H-(b-Ala)-Sar₁₀-Ala-Cys-(D-Ala)-Asn-Glu-(1Nal)-(D-Ala)-Cys-Glu-Asp-Phe-Tyr-Asp-(tBuGly)-Cys-NH2 (SEQ ID NO: 5);
or
Acetyl-(b-Ala)-Sar₁₀-Ala-Cys-(D-Ala)-Asn-Glu-(1Nal)-(D-Ala)-Cys-Glu-Asp-Phe-Tyr-Asp-(tBuGly)-Cys-NH2 (SEQ ID NO:); or
H-Ala-Cys-(D-Ala)-Asn-Glu-(1Nal)-(D-Ala)-Cys-Glu-Asp-Phe-(4BrPhe)-Asp-(tBuGly)-Cys-NH2 (SEQ ID NO:),
wherein Sar is sarcosine, where D-Ala is D-Alanine, where 1NAl is 1-naphthyl-L-alanine, where tBuGly is α-t-butylglycine and where b-Ala is β-Alanine.

11. A compound according to any preceding claim, wherein the looped peptide structure is attached to the scaffold via at least three sulfone linkages to form a bicyclic structure on the scaffold.

12. A compound according to any preceding claim, wherein the scaffold comprises an (hetero)aromatic or (hetero)alicyclic moiety.

13. A compound according to claim 12, wherein the scaffold comprises a *tris-*substituted six-membered ring structure, preferably wherein the scaffold has a 3-fold symmetry axis.

14. A compound according to claim 13, wherein the scaffold comprises *tris*-methylene (hetero)aryl, preferably wherein the scaffold moiety is 1,3,5-*tris*-methylene phenyl.

15. A method of making a compound according to any preceding claim, the method comprising:
providing a peptide having at least two cysteine residues;
providing a scaffold molecule having at least two reactive sites for forming thioether linkages with the cysteine residues;
forming said thioether linkages between the peptide and the scaffold molecule; and
oxidizing the thioether linkages to sulfone linkages.

16. A compound comprising at least one looped peptide structure attached via at least two sulfone linkages formed through cysteine residues of the peptide to a scaffold, wherein the Kd of the compound towards a predetermined target molecule is no more than about 20 times the Kd of the corresponding compound having thioether linkages in place of the sulfone linkages.
